# EUROPEAN PATENT APPLICATION

(11) **EP 2 578 308 A2**
(43) Date of publication of application: **10.04.2013**
(21) Application number: 12187288.1
(22) Date of filing: 04.10.2012
(51) Int. Cl.: B01F 13/00, B01F 15/02, B01F 7/18, A61B 17/88

(54) **An apparatus and a process for preparation of bone cements**

(30) Priority: 04.10.2011 IT MI20111798
(71) Applicant: G21 S.r.l., 41039 San Possidonio (MO) (IT)
(72) Inventor: Vaccari, Alberto, I-41126 Modena (IT); Foroni, Filippo, I-41037 Mirandola (MO) (IT)
(74) Representative: Sutto, Luca

(57) **Abstract**

An apparatus for preparation of bone cements (1), comprising: a mixer device (2) having: a housing body (3) defining a chamber (4), housing or able to house a predetermined quantity of a solid component (5), for example in a granular or powder form, at least an inlet port (6) for enabling access of at least a fluid towards the chamber (4); the apparatus (1) further comprising a check and closing valve (7) operating in proximity of or at the inlet port (6) and being predisposed to selectively open and close the fluid passage towards the chamber (4) of the housing body (3).

## Description

The present invention relates to an apparatus typically, though not necessarily, of a single-use disposable type, for preparation of bone cements. The invention also concerns a method for preparing bone cements, in particular using the apparatus of the invention.

The bone cements prepared with the method and apparatus described herein can, for example, be used for surgical operations in arthroplasty procedures such as those concerning the hip, knee and other joints, in connection with joint prosthesis, bone reconstruction, bone structure consolidation procedures and the like.

### Background of the Invention

As is known, polymeric-based bone cements, and in particular acrylic, i.e. having an organic or inorganic matrix at present in use are typically constituted by two or more components, among which: a liquid component, which in the case of the acrylic cement comprises a volatile monomer, and a solid component, typically in powder form. The liquid component and the solid component are mixed such as to form the bone cement which, later, is to be used rather quickly so that the solidification occurs with the cement already positioned in the desired sites internally of the body. Note that the liquid component, in the case of acrylic-based cement comprising metacrylate, is characterised by a pungent odour, has characteristics of inflammability and in general is dangerous to touch. The liquid component is typically housed in a glass vial which at the moment of use is opened by breakage and then added to and mixed, carefully, with the solid component.

The mixing devices at present in use are of various types.

A first solution includes the use of a bowl or a mixer device into which the liquid and solid components are conveyed, with the aim of enabling mixing thereof before dispensing into the bone tissue to be treated.

A second technical solution includes use of a mechanical-type mixer, in which the liquid component and the solid component must be poured manually, after opening the primary containers of the components and of the mixer.

A third technical solution, such as for example the one described in US publication 2003/0067837A1, includes use of a mixing chamber provided with a mixer activatable from outside in which a predetermined load of the solid component in powder form is housed. At an inlet port of the mixing chamber a perforable wall is located, which can cooperate with a double spike destined on one side to perforate the wall present on the above-described port, and on the other side to perforate a further wall present on a vial in which the liquid component is housed.

In practice the double spike serves to perforate the respective walls present on the vial and on the port of the mixing chamber, such as to enable transferring the liquid component into the chamber where the solid component is housed. Although this solution is an improvement with respect to the previously-described one, as it ensures a certain degree of protection for the user and a certain degree of protection of sterility, it is however worthy of note that the use of the perforable walls can lead to sealing problems, especially when the perforation devices are removed from the walls, and a smaller flow of fluid during the passage from the vial to the mixing chamber, as well as problems of repeatability, constructional accuracy, and very significant residual risks for the operator.

### Summary of the Invention

In this situation, an aim at the base of the present invention is to disclose an apparatus and a method for preparing bone cements which enable maximising the ergonomics and simplicity of use thereof, repeatability of the optimum process conditions, maintaining the conditions of sterility, protection from chemical contamination from the environment following use of volatile substances, powders which can disperse in the air or accidental spreading, and excluding the potential contamination of the cement deriving from solid, liquid, gaseous substances as well as air-transported powders or other sources of chemical and biological contamination present in the work environment, thus obtaining maximum safety for the operator preparing the cement, and for the other persons present in the use environment, as well as the maximum guarantee of adequacy and safety of the cement for the patient it is destined for.

A further aim of the invention is to make available a device that is typically but not necessarily of a single-use disposable type, easy to realise and which enable production and setting-up which are relatively inexpensive.

A further aim of the invention is to provide a device which can house the components of the bone cement preloaded during the production step, which then enables preventing the initial steps of opening the primary containers and transferring and filling in the mixing means in the operating theatre and thus steps which are potentially at risk of microbiologial contamination of the cement and environmental as regards the exposing of the operators to potentially toxic chemical agents.

A further aim of the invention is to disclose a device and a method which enable carrying out the passage of the liquid component towards the solid component within a closed system, in short times and without fluid seal problems.

Lastly, an aim of the invention is to provide an apparatus and a method which can be respectively used and set up intuitively and easily by the operator.

At least one of the above aims is substantially attained by an apparatus and a method according to one or more of the appended claims.

A first aspect relates to an apparatus for preparation of bone cements (1), comprising: a mixer device (2) having: a housing body (3) defining a chamber (4), housing or able to house a predetermined quantity of a solid component (5), for example in a granular or powder form, at least an inlet port (6) for enabling access of at least a fluid towards the chamber (4); the apparatus further comprising a check and closing valve (7) operating in proximity of or at the inlet port (6) and being predisposed to selectively open and close the fluid passage towards the chamber (4) of the housing body (3).

In a 2nd aspect according to the preceding aspect the apparatus comprises at least a container (8) housing a predetermined quantity of a liquid component (9) and removably engageable to the inlet port (6).

In a 3rd aspect according to the preceding aspect, the container comprises at least an outlet opening (10) at which a closing organ (11) operates.

In a 4th aspect according to any one of the preceding aspects, the apparatus comprises a tubular body (12) housing the valve (7) internally thereof and having a first terminal portion (12a) engaged to the inlet port (6) and a second terminal portion (12b) facing away from the housing body (3) of the mixer device (2) and defining an engaging collar (13).

In a 5th aspect according to the preceding aspect, the apparatus comprises a transfer adapter (14) having a shank (15) able to slidably engage with the engaging collar (13) of the tubular body.

In a 6th aspect according to the preceding aspect, the transfer adapter (14) is able to displace in the tubular body up to an operating position in which the shank (15) acts to open the valve.

In a 7th aspect according to aspects 5 or 6, the transfer adapter (14) comprises, in an axially opposite position to the shank (15), a removable coupling portion (16) with the container (8) of the liquid component, the coupling portion (16) being configured such as to act on the closing organ (11) and to determine the opening of the fluid passage through the outlet opening (10) when the coupling portion (16) engages with the container (8) of the liquid component (9).

In an 8th aspect, the closing organ (11) comprises a perforable membrane (17) sealing the outlet opening of the container of the liquid component.

In a 9th aspect according to the preceding aspect, the coupling portion (16) of the transfer adapter (14) comprises a perforating element (18) able to pass through the perforable membrane (17) when the coupling portion engages with the container (8) of the liquid component.

In a 10th aspect according to aspects from 7 to 9, the shank (15) comprises an internal channel (19) axially crossing the shank and in communication with a further channel (20) crossing the coupling portion (16).

In an 11th aspect according to the preceding aspect, the channel (20) internally crossing the perforating element.

In a 12the aspect according to any one of aspects from 4 to 11, the first terminal portion (12a) of the tubular body (12) is configured for removably coupling to the inlet portion (6) of the mixer device.

In a 13th aspect according to any one of the preceding aspects the valve comprises an elastic thrust element (21) housed in the tubular body.

In a 14th aspect according to the preceding aspect, the valve comprises an obturator (22) destined to operate to close the fluid passage across the tubular body (12), by acting to abut, upon a thrust of the elastic element, against a seal edge (23) predisposed on an internal surface of the tubular body (12).

In a 15th aspect according to the preceding aspect, the elastic element (21), for example comprising at least a helix spring, exhibits a first end (21a) that acts on the obturator (22) and a second end (21b) that acts on an abutment (24) that is axially solid with the tubular body.

In a 16th aspect according to the preceding aspect, the elastic element (21) comprises a spring.

In a 17th aspect according to aspects 15 or 16, the elastic element is normally compressed such as to exert an elastic thrust action on the obturator.

In an 18th aspect according to aspects from 13 to 17, the shank (15) of the transfer adapter (14) is removably couplable to the tubular body (12) and is displaceable up to the operating position in which the shank (15) acts to push the obturator (22), opening a fluid passage between the internal channel (20) of the perforating element and the channel (19) crossing the shank and thus setting the internal channel (20) and the perforating element in communication with the inlet port (6) of the chamber (4) of the mixer device.

In a 19th aspect according to any one of aspects from 5 to 18, the shank (15) comprises a radially external surface (15a) of prismatic conformation, i.e. cylindrical, capable of slidably joining with an internal surface (12c) of the tubular body (12).

In a 20th aspect according to the preceding aspect, the radially external surface (15a) defining at least an annular seating (25) in which a seal element (25a), also having an annular conformation, operates, the seal element (25a) being positioned between the radially external surface of the shank and the radially internal surface of the tubular body when the shank is inserted and made to slide in the tubular body.

In a 21st aspect according to any one of the preceding aspects, the apparatus comprises at least a mixer organ (26) having a stem (27) exhibiting a manoeuvring portion (28) emerging from the housing body and an active portion (29) housed internally of the chamber and having one or more radial mixer elements (30).

In a 22nd aspect according to the preceding aspect, the manoeuvring portion (28) comprises at least a joint (31) for enabling angular bending of a terminal part of the stem with respect to the active part.

In a 23rd aspect according to the preceding aspect, the terminal part further optionally bearing a gripping handle (32).

In a 24th aspect according to any one of the preceding aspects, the housing body (3) comprises a tubular main body (33) having an end to which a cover (34) is sealingly fixed.

In a 25th aspect according to the preceding aspect, the cover (34) in turn exhibits the inlet port (6), an aspirating port (35) connectable to a vacuum line and a through-hole (37) for receiving a stem (27) of a mixer organ (26).

In a 26th aspect according to aspects 24 or 25, the housing body (3), on an opposite side to the cover (34), comprises a terminal closing element (38) sealedly fixed to the main body.

In a 27th aspect according to any one of the preceding aspects, the housing body (3) is made of a plastic material.

In a 28th aspect according to any one of aspects from 24 to 27, the main body (33) is made of an optically-transparent or semi-transparent plastic material.

In a 29th aspect according to any one of aspects from 4 to 28, the tubular body (12) housing the valve (7) is unremovably joined to the inlet port.

In a 30th aspect, an apparatus is comprised for preparing bone cements, optionally according to any one of the preceding claims, comprising a mixer device (2) having: a housing body (3) defining a chamber (4) housing or able to house a predetermined quantity of a solid component (5), for example in granular or powder form, at least an inlet port (6) for enabling access of fluid towards the chamber (4), at least a mixer organ (26); the mixer organ (26) having: a stem (27) provided with a manoeuvring portion (28) emerging from the housing body and an active portion (29) internally housed in the chamber (4), a joint (70) comprising a first segment (71) and a second segment (72) rotatably engaged to one another, and wherein the first segment has a first end (71a) connected to the active portion (29) and a second end (71b) which cooperates with the second end (72b) of the second segment (72) such as to define a hinge, for example a flat hinge, the second segment (72) being connected to the manoeuvring portion (28), and a holding element (76), for example in a form of a tubular sleeve, which in a first operating position internally receives the joint (50) and maintains the two segments (71 and 72) in aligned conditions, and in a second operating condition, distanced axially or angularly with respect to the first operating position, frees the joint (50) and enables angular inclination of the first segment with respect to the second segment.

In a 31 st aspect according to the preceding aspect the sleeve (76) exhibits a first prismatic or cylindrical seating (77), complementarily shaped to the external surface of the two segments (71 and 72) and a second prismatic or cylindrical seating (78) consecutive to and having a greater diameter than the first, which second sliding seating houses the elastic element (79) which in turn exhibits a first end (79a) acting abuttingly against an edge (80) solidly constrained to the manoeuvring portion (28) and a second end (79b) acting abuttingly against a connecting wall (81) between the first and the second seating.

In a 32nd aspect according to aspects 30 or 31, a radial lip (82) solidly constrained to the first segment and emerging from an external lateral surface thereof delimits the axial run of the sleeve with respect to the two segments.

In a 33rd aspect a process is comprised for forming bone cements using an apparatus as in any one of the preceding claims, comprising steps of:
a. connecting the chamber (4) of the mixer device (2) to an aspirating system for generating a predetermined depression level in the chamber;
b. in a case in which the tubular body (12) is a separate component from the inlet port (6), engaging the tubular body to the inlet port;
c. inserting the transfer adapter (14) at the free end of the tubular body (12);
d. inserting at least the portion of the container (8) of the liquid component delimiting the outlet opening at the coupling portion (16) of the transfer adapter (14) such as to determine an opening of a passage of fluid through the outlet opening;
e. axially pushing the container (8) of the liquid component towards the transfer adapter (14), in such a way that the shank (15) of the adapter can, in turn, act to push on the obturator (22) of the valve (100) and thus open a fluid passage from the container of the liquid component up to the chamber of the mixer device;
f. maintaining the pushed condition for a predetermined time, for example at least 10 seconds, enabling the liquid component to enter the chamber (4) of the mixer device containing the solid component;
g. removing the container (8) of the liquid component and the transfer adapter (14) from the tubular body (12) such as to enable the valve (100) to be closed;
h. mixing for a predetermined time, up to formation of the bone cement.

In a 34th aspect according to the preceding aspect, the process can comprise a substep of removing a protection cover arranged on the transfer adapter before proceeding to inserting the portion of the container on the coupling portion of the transfer adapter.

In a 35th aspect according to aspects 33 or 34, the process comprises a substep comprising the extraction of the manoeuvring portion (38) from the chamber (4).

In a 36th aspect according to the preceding aspect the substep of extraction of the manoeuvring portion comprises a substep comprising exit of a facilitated easy-break segment (52) from the chamber (4) through the opening (37).

In a 37th aspect according to the preceding aspect, the process comprises a substep comprising the inclination of the manoeuvring portion out of the axis thereof such as to cause breakage and expansion of the segment (52) and all the manoeuvring part is removed (28).

In a 38th aspect according to any one of aspects from 33 to 37, the process comprises a substep which comprises the separation of the valve-fitted tube (12) from the port (6) to which a dispensing cannula is applied.

In a 39th aspect according to any one of aspects from 33 to 38, the process comprises a substep which comprises coupling the container (3) to a conventional pistol, which for example acts pushingly on the piston (138) to promote exit of the cement from the port (6).

A 40th aspect comprises an apparatus for preparation of bone cements (1), comprising:
a mixer device (2) having:
   a housing body (3) defining a chamber (4), housing or able to house a predetermined quantity of a solid component (5), for example in a granular or powder form,
   at least an inlet port (6) for enabling access of at least a fluid towards the chamber (4);
   a check and closing valve (7) operating in proximity of or at the inlet port (6), the check and closing valve (7) comprising a valve body (101) defining a fluid passage channel (102) and an obturator organ (22) cooperating with the valve body (101) and displaceable between a closed condition, in which it intercepts the fluid passage across the channel (102) and an open condition, in which it enables fluid passage through the channel (102), the valve (100) being predisposed to selectively open and close the passage of fluid towards the chamber (4) of the housing body (3).

In a 41st aspect according to aspect 40, the apparatus further comprises at least a container (8) housing a predetermined quantity of a liquid component (9) and comprising at least an outlet opening (10) at which a closing organ (11) operates.

In a 42nd aspect according to aspect 41, the apparatus further comprises at least a transfer adapter (14) removably engageable on a side to the container (8) and on an opposite side, to the inlet port (6), the transfer adapter, when engaged to the container (8) and the door (6), acting to open both the closing organ (11) and to push the obturator organ (22), opening the valve (100).

Ina 43rd aspect according to any one of aspects from 40 to 42, the apparatus comprises a tubular body (12) defining the valve body (101) and having a first terminal portion (12a) engaged to the inlet port (6) and a second terminal portion (12b) facing away from the housing body (3) of the mixer device (2) and defining an engaging collar (13).

In a 44th aspect according to any one of aspects from 41 to 43, the transfer adapter (14) exhibits a shank (15) able to slidably engage with the engaging collar (13) of the valve body and able to displace in the valve body up to an operating position in which the shank (15) acts to open the valve.

In a 45th aspect according to any one of aspects from 41 to 44, the transfer adapter (14) exhibits a shank (15) able to slidably engage with the valve body (101), in particular with the engaging collar (13) of the valve body, and to displace in the valve body up to an operating position in which the shank (15) acts to open the valve.

In a 46th aspect according to any one of aspects from 41 to 45, the transfer adapter (14) comprises, in an axially opposite position to the shank (15), a removable coupling portion (16) with the container (8) of the liquid component, the coupling portion (16) being configured such as to act on the closing organ (11) and to determine the opening of the fluid passage through the outlet opening (10) when the coupling portion (16) engages with the container (8) of the liquid component (9).

In a 47th aspect according to any one of aspects from 41 to 46, the closing organ (11) comprises a perforable membrane (17) sealing the outlet opening of the container of the liquid component, and wherein the coupling portion (16) of the transfer adapter (14) comprises a perforating element (18) able to pass through the perforable membrane (17) when the coupling portion engages with the container (8) of the liquid component.

In a 48th aspect according to any one of aspects from 45 to 47, the shank (15) comprises an internal channel (19) axially crossing the shank and in communication with a further channel (20) crossing the coupling portion (16), in particular internally crossing the perforating element.

In a 49th aspect according to any one of aspects from 40 to 48, the valve (100) comprises an elastic thrust element (21) housed in the valve body and an obturator (22) destined to operate to close the fluid passage across the valve body (12), by acting to abut, upon a thrust of the elastic element, against a seal edge (23) predisposed on an internal surface of the valve body (101).

In a 50th aspect according to the preceding aspect the elastic element (21), for example comprises at least a helix spring, exhibits a first end (21a) that acts on the obturator (22) and a second end (21b) that acts on an abutment (24) that is axially solid with the valve body (101), the elastic element being normally compressed such as to exert an elastic thrust action on the obturator that is sufficient to maintain the obturator in the closed position even in the presence of a difference between the pressure existing internally of the chamber (4) and the ambient atmospheric pressure existing externally of the housing body (3).

For example the thrust exerted by the spring can maintain the obturator in hermetic closure even in a case of pressure difference between the inside and outside of the housing body of greater than 0.3 bar, or 0.5 bar, or greater than 0.8 bar.

In a 51st aspect according to any one of aspects from 45 to 50, the shank (15) of the transfer adapter (14) is removably couplable to the tubular body (12) and is displaceable up to the operating position in which the shank (15) acts to push the obturator (22), opening a fluid passage between the internal channel (20) of the perforating element and the channel (19) crossing the shank and thus setting the internal channel (20) and the perforating element in communication with the inlet port (6) of the chamber (4) of the mixer device.

In a 52nd aspect according to any one of aspects from 45 to 51, the shank (15) comprises a radially external surface (15a) of prismatic conformation, i.e. cylindrical, capable of slidably joining with an internal surface (12c) of the valve body, in particular with an internal surface (12c) of the tubular body (12), the radially external surface (15a) defining at least an annular seating (25) in which a seal element (25a), also having an annular conformation, operates, the seal element (25a) being positioned between the radially external surface of the shank and the radially internal surface of the valve body when the shank is inserted and made to slide in the valve body.

In a 53rd aspect according to any one of aspects from 41 to 53, the container (8) of the liquid component (9) comprises a containing element having an at least partially deformable portion, for example axially deformable, such that the internal housing volume of the liquid component (9) can progressively reduce as the liquid component is extracted from the container (8).

In a 54th aspect according to the preceding aspect the container (8) comprises at least one of the following:
- a containing element constituted by a bag made of a plastic material which collapses on itself progressively as the liquid component is extracted,
- a containing element constituted by a bag made of a plastic material having a bellows portion that is axially collapsible progressively as the liquid is extracted,
- a containing element having a lateral wall and a terminal wall, opposite the opening, and slidable axially and sealedly along the lateral wall progressively nearingly to the opening as the liquid component is extracted.

Practically, the housing body (3) can be subjected to depression through a port, different to the port (6) to which an aspirating system is connectable. The container is advantageously configured such as to collapse progressively and thus to reduce its internal volume, enabling the liquid to freely flow out.

In a 55th aspect according to any one of aspects from 40 to 54, the apparatus comprises at least a mixer organ (26) having a stem (27) provided with a manoeuvring portion (28) emerging externally of the housing body and an active portion (29) internally housed in the chamber (4).

In a 56th aspect according to the preceding aspect, the active portion (29) comprises one or more radial mixing elements (30).

In a 57th aspect according to aspects 55 or 56, the manoeuvring portion (28) comprises at least a joint (31) for enabling angularly bending a terminal part of the stem with respect to the active portion, the terminal part further optionally bearing a gripping handle (32).

In a 58th aspect according to any one of aspects from 40 to 57, the housing body (3) comprises a tubular main body (33) having an end to which a cover (34) is sealingly fixed, which cover (34) in turn exhibits the inlet port (6), an aspirating port (35) connectable to a vacuum line and a through-hole (37) for receiving a stem (27) of a mixer organ (26).

In a 59th aspect according to the preceding aspect the housing body (3), on an opposite side to the cover (34), comprises a terminal closing element (38) sealedly fixed to the main body.

In a 60th aspect according to any one of aspects from 40 to 59, the housing body (3) is made of a plastic material and wherein the main body (33) is made of an optically-transparent or semi-transparent plastic material.

In a 61st aspect according to any one of aspects from 40 to 60, the valve body, optionally the tubular body (12), is unremovably joined to the inlet port.

In a 62nd aspect a process is comprised for forming bone cements using an apparatus according to any one of aspects from 40 to 61, the process comprising steps of:
a. connecting the chamber (4) of the mixer device (2) to an aspirating system for generating a predetermined depression level in the chamber;
b. in a case in which the valve body (12) is a separate component from the inlet port (6), engaging the valve body to the inlet port;
c. coupling the transfer adapter (14) to the valve body (12);
d. inserting at least the portion of the container (8) of the liquid component delimiting the outlet opening at the coupling portion (16) of the transfer adapter (14) such as to determine an opening of a passage of fluid through the outlet opening;
e. axially pushing the container (8) of the liquid component towards the transfer adapter (14), in such a way that the shank (15) of the adapter can, in turn, act to push on the obturator (22) of the valve (100) and thus open a fluid passage from the container of the liquid component up to the chamber of the mixer device;
f. maintaining the pushed condition for a predetermined time, for example at least 10 seconds, enabling the liquid component to enter the chamber (4) of the mixer device containing the solid component;
g. removing the container (8) of the liquid component and the transfer adapter (14) from the tubular body (12) such as to enable the valve (7) to be closed;
h. mixing for a predetermined time, up to formation of the bone cement.

Following step h, the bone cement can be dispensed from a port that is different to the port 6 and which is also borne by or present in the body (3).

Further aspects of the invention are described herein below, with reference to the accompanying figures supplied purely by way of non-limiting example:
figure 1 shows a perspective view of the assembly of a mixing device forming part of the apparatus of preparation of bone cements according to the invention;
figure 2 shows a perspective view of the mixing device of figure 1 to which a container of a liquid component is applied after interposition of a valved tubular body and a transfer adapter;
figures 2A, 2B, 2C, 2D show a container of the liquid component respectively with bellows, defined by a bag, with an end wall axially displaceable or entirely rigid (vial);
figures 3A and 3B shows, in longitudinal section, the components forming the valved tubular body of figure 2 respectively under conditions of closed valve and open valve.
figure 4 shows, in side elevation, an example of a transfer adapter coupled to the valved tubular body of figure 3;
figure 5 is a section along the line V-V of figure 4;
figure 6 is a plan view of the adapter of figure 4;
figure 7 is a perspective view of the adapter of figure 4;
figure 8 shows a longitudinal section of a mixing member in accordance with an aspect of the invention;
figure 9 shows a lateral view of a portion of the organ shown in figure 8;
figure 10 is a perspective view relating to a first segment of the stem of the organ mixer of figure 8;
figure 11 is a side view of the first segment shown in figure 10;
figure 12 is a front view of the first segment shown in figure 10;
figure 13 is a section along line XIII-XIII shown in figure 12;
figure 14 is a plan view of the first segment shown in figure 10;
figure 15 is a perspective view of a second segment of the rod of the mixer body of figure 8;
figure 16 is a side view of the second segment shown in figure 15;
figure 17 is a front view of the second segment shown in figure 15;
figure 18 is a longitudinal section of the sleeve which at least partially accommodates the first and the second segment of the rod of the mixer body.

### Detailed description

With reference to the accompanying drawings, below some embodiments in accordance with the invention are described. Obviously variants can be provided to the embodiments described herein, provided they are within the scope of protection as defined in one or more of the appended claims.

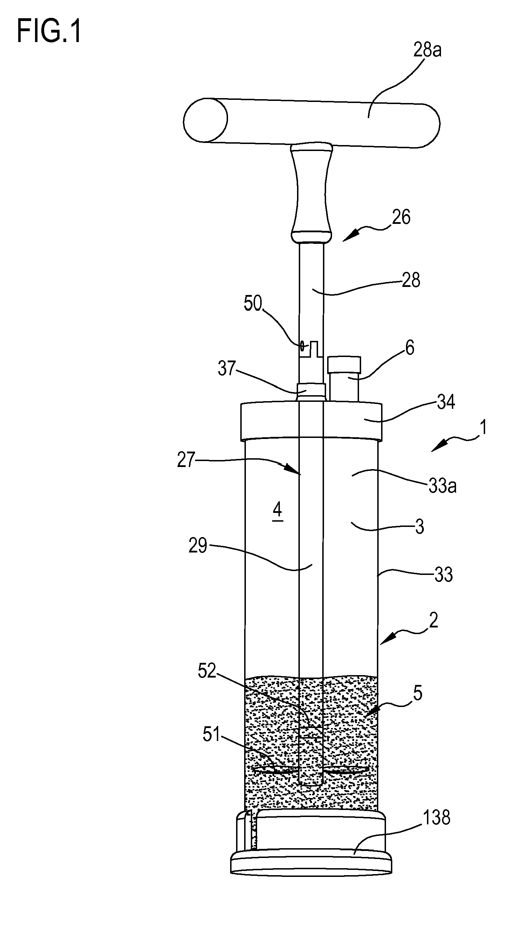

With reference to figure 1, reference numeral 1 denotes in its entirety an apparatus for preparation of bone cements, in accordance with aspects of the invention. The apparatus 1 comprises a mixing device 2 having a housing body 3, internally defining a chamber 4 which houses a predetermined load of a solid component 5, for example in granular or in powder form. The chemical matrix of the solid component can comprise one or more of the following ingredients: polymers, radiopaque elements, reaction catalysts, drugs with ancillary function, inert or bioactive materials.

In greater structural detail, the housing body 3 may comprise a tubular main body 33, having an end 33A to which a cover 34 is sealingly fixed. The cover 34 has an inlet port 6 to the chamber 4. As will become apparent hereinafter, the entrance port 6 allows access of fluid to the chamber 4 during the preparation of the bone cement. In a further embodiment, not shown in the accompanying figures, the lid 34 can comprise a plurality of inlet ports 6, each of which allows the entry of a fluid into the chamber 4. In this way, from the input ports 6, access to the chamber 4 at least one type of fluid into the chamber can be afforded, i.e. the release of two or more types of liquid.

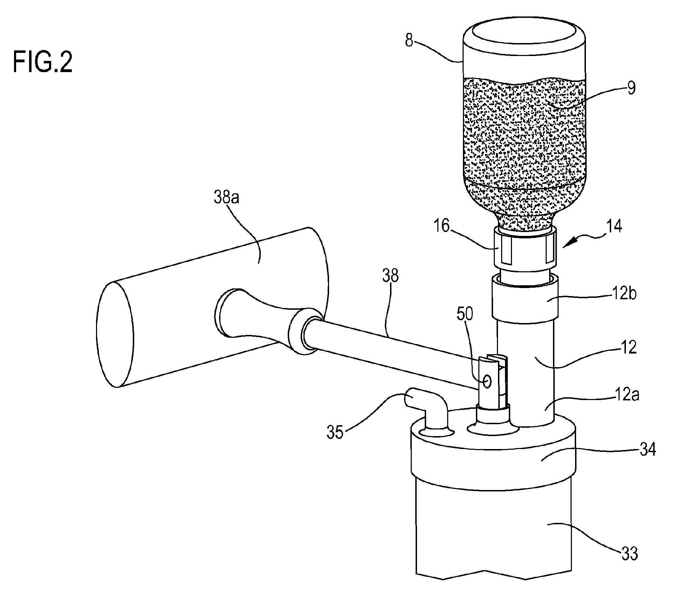

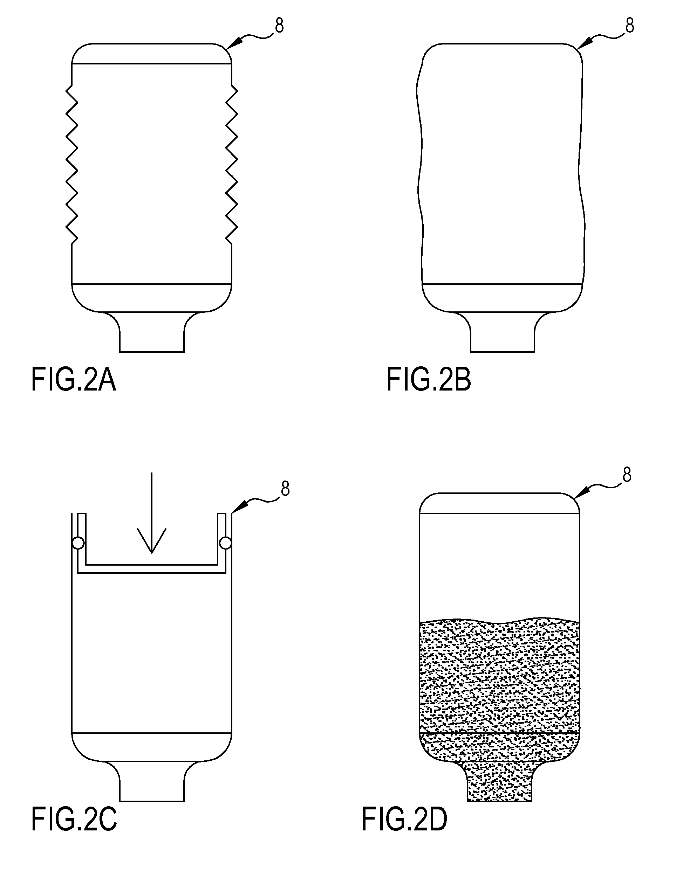

The lid 34 may also present an aspirating port 35 (figure 2) connectable to a vacuum line or to a suction line (not represented as known *per se*)*,* capable of creating a predetermined level of depression inside the chamber 4 that is conducive to proper mixing and degassing of the solid component 5 with the liquid component, thus avoiding the formation of bubbles embedded in the rubber composition which could affect the mechanical performance of the bone cement. A terminal element 138 is also provided inferiorly on the side opposite the cover, which acts to close the tubular main body 33, so as to realise a chamber 4 which essentially communicates with the outside through the entrance port 6 and through the aspirating port 35. The end element 138 can comprise a piston, sealingly slidable in the chamber 4, such as to enable the delivery of the cement after completion of the mixing.

At least a container 8 can be constrained to the entrance door 6, housing a predetermined load of a liquid component 9. The container (8) can be a rigid container or a vial, of the type illustrated in figure 2D which is in this case only partially filled with liquid. Alternatively, the container (8) can comprise a containing element (figure 2B) constituted by a bag made of a plastic material which progressively collapses on itself as the liquid component is extracted, or a containing element made of plastic material having a axially collapsible bellows portion (figure 2A) as the liquid component is extracted, or a containing element having a lateral wall for example, prismatic or cylindrical and an end wall, opposite the opening, and axially slidable and sealed along the lateral wall in a nearing direction to the opening as the liquid component is extracted (figure 2C).

In the case of figure 2C, the container 8 can alternatively be a syringe: in this case the mobile terminal wall is formed by the terminal part of a piston sealingly slidable internally of a syringe body which bears, on the side opposite to the piston, engagement means to the transfer body 14 (described below) or means for direct connection to the valve body 101. In the case where the syringe engages the transfer body, it terminally comprises a collar 14 housing a perforable wall 11 which can be perforated by the perforating element 18 of the transfer body 14. In the case where the syringe engages to or is connected to the valve body 101 of the valve 100, the terminal part of the syringe can exhibit a thrust shank for opening the obturator, for example similar to the shank 15 of the element 14. Further, in the case where the syringe engages or is connected to the valve body 101 of the valve 100, it can either be removably attached or non-removably attached: in the first case the dispensing area of the syringe is preferably protected with a removable sealing means.

In practice, the housing body 3 can be subjected to a depression through a port, distinct from the port 6 to which a suction system is connectable. In the configurations of figures 2A-2C, the container is advantageously configured such as to progressively collapse (or to reduce its internal volume thanks to the translation of a wall or a plunger - figure 2C) and thereby to reduce the volume available for the liquid housed inside the container, enabling the liquid itself to exit freely.

The container 8, once engaged directly or indirectly to the front door, enables the transfer of the liquid component into the chamber 4, so that liquid component and solid component are in the same environment. As previously described, the cover 34 may comprise a plurality of input ports 6: in this situation the apparatus 1 can comprise a plurality of containers 8, one for each input port 6 (condition not represented in the accompanying figures). To better detail the last condition described, the containers 8 can accommodate the same liquid solution or alternatively accommodate different solutions that allow mixing with the solid component present within the chamber 4.

Continuing with the description of the apparatus 1, it can be seen from the accompanying figures that the apparatus includes, but is not limited to, a mixer organ 26 equipped with a stem 27 and having a manoeuvring portion 28 which, as can be seen in the accompanying figures, emerges from the housing body 3, for example through a through-opening 37 arranged on the cover 34. In other words, the stem 27 passes through the through-opening 37 and is free to move vertically and rotate around its own axis upon the action exerted by the user on the manoeuvring portion 28. In the illustrated embodiment, for example in figure 1, the manoeuvring portion 28 comprises a terminal handle 28a and an articulated joint 50 which enables angularly bending an end portion of the stem 27 with respect to an active portion 29 of the stem itself which is housed internally of the chamber 4. The active portion 29 operating inside the chamber 4 also exhibits one or more radial elements 51, for example blade-shaped which, by rotating the mixer 26, enable it to actuate the mixing of the liquid component with the solid component. Note that since the housing body 3 is sealedly coupled both to the cover 34 and the terminal element 38, and since the stem 27 passes through the through-opening 37 while still guaranteeing the fluid-tight seal, during the mixing step there is no risk that any liquid material and/or powders can come into contact with the user.

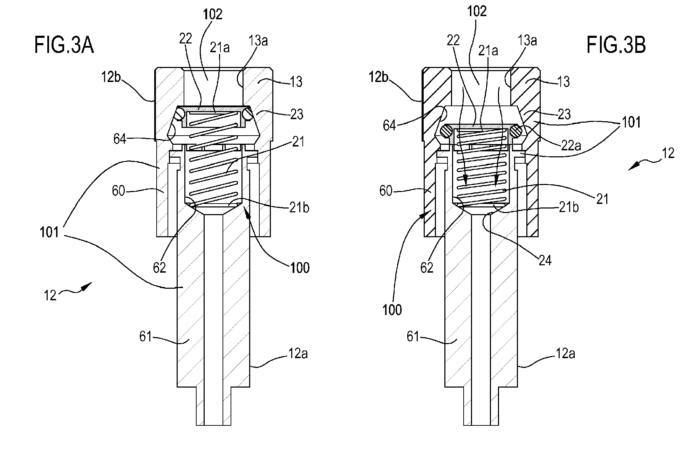

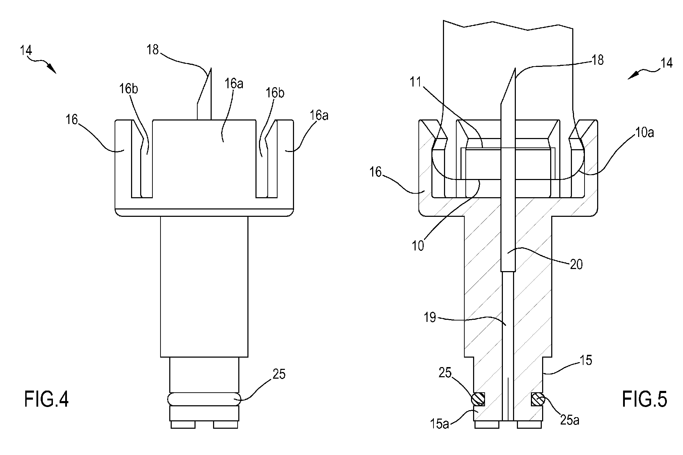

In accordance with an aspect of the invention, a tubular body 12 operates at the inlet port 6 and exhibits (see figures 3A and 3B) a first terminal portion 12a constrained or constrainable to the housing body at the inlet port 6, and a second end portion 12b facing away from the housing body 3 of the mixer device 2. The second end portion 12b also defines an engaging collar 13 for receiving a shank 15 of a transfer adapter 14, the structural details of which can be as illustrated in the non-limiting example of figures 4-7. More precisely, the transfer adapter 14, which in the illustrated embodiment has an axialsymmetric structure, comprises a shank 15 which, as already mentioned, can couple to the engaging collar 13 of the tubular body 12 and, on the axially opposite side to the shank 15, a coupling portion 16 capable of removably engaging with at least a terminal edge 10a delimiting the outlet opening 10 of the container 8 housing the liquid component 9. In practice, the shank 15 of the transfer adapter 14 couples slidingly with the engagement collar 13 of the tubular body and is capable of moving internally of the tubular body up to an operative position in which the shank opens the obturator 22 of a check valve 100 (figure 3B). On the side axially opposite the shank 15, the coupling portion 16 is configured to act on the closing organ 11 borne by the opening 10 of the container 8 such as to determine the opening of the closure member 11 and cause fluid passage through the outlet opening, when the coupling portion 16 engages the edge 10a of the container 8.

In the embodiment illustrated in figure 5, the closing member 11 can include a perforable wall, made for example by the use of an elastomer material, which under normal conditions seals the outlet opening of the container 8. The wall can be pierced by a needle or a cannula or other piercing element 18 borne by the coupling portion 16 of the transfer adapter 14. More precisely, when the coupling portion 16 receives the edge 10a, the piercing element 18 is inserted through the wall 11, creating a fluid communication between the inside of the container 8 and a channel 20 inside the piercing element 18. As can be seen from figure 5, the shank 15 comprises an internal channel 19 extending through the shank itself and in fluid communication with the channel 20 crossing through the coupling portion 16 and in particular, passing through the piercing element 18. At the moment in which the piercing element 18 passes through the perforable wall 11, the fluid contained in the container 8 can pass through the channels 19 and 20 up to reaching the inside of the shank 15.

As mentioned, the shank 15 is configured to insert into the tubular body 12 and bring the valve 100 into the open condition, so that the fluid passing through the channels 19 and 20 can be dispensed through the port 6 in the chamber 4.

In greater detail, and again referring to figures 3A and 3B, it can be noted that the tubular body 12 can for example be realized in several parts: a ring nut 60 can for example define the second end portion 12b and the collar 13; the ring nut 60 engages with a tube 61 so as to be at least axially constrained therewith (the constraint can for example be achieved with a snap fit, bayonet, screw-nut screw coupling or another besides). The tube 61 has at one end the first end portion 12a and at a second end, facing the ring nut 60, a seating 62. The valve 100 defined in the tubular body 12 comprises an elastic thrust element 21, for example comprising at least a helical spring, housed in the tubular body 12 and thrustingly acting on the obturator 22, for example plate-shaped, destined to operate to close the passage of fluid through the tubular body 12. In practice, a first end 21a of the elastic element 21 pushes the obturator 22 towards the ring nut 60 and a second end 21 b is received in the seating 62 which acts abuttingly on the bottom of the seating, which is solidly constrained with the tubular body 12; alternatively the second end 21b acts on an abutment 24 of the tubular body 12. In more detail, a peripheral edge of the obturator 22 houses a sealing element 22a which acts in abutment on a conical surface 64 of a sealing edge 23; the conical surface 64 extends in a radially internal position to the nut 60 and diverges away from the collar 13. In practice, when the shank 15 is not inserted in the tubular body 12, the obturator 22 acts in abutment, on the thrust of the elastic element 21, against the surface 64 of the sealing flange 23 disposed inside the tubular body 12 (figure 3A). When, conversely, the shank 15 acts thrustingly on the obturator 22 against the force exerted by the elastic thrust 21 (figure 3B), a passage of fluid opens from the channel 19 towards the port 6. In the example shown in figure 3B, the fluid passage is realized thanks to the presence of terminal projections carried by the tube 61 which receive in abutment a lower edge of the obturator and which form therewith one or more windows of the fluid passage towards the seating 62. Since the elastic element 21 has the second end 21b active on the obturator 22 and the first end 21a active against the abutment formed by the bottom of the seating 62, and since this elastic element 21 is normally in a state of compression, an elastic thrust action is exerted on the obturator such as to maintain the obturator in the closed position of the check valve 100, unless there are actions exerted by the shank 15. Practically, when the transfer adapter 14 is not engaged with the tubular body the check valve is normally closed, isolating the chamber 4 from the outside environment. More generally, the valve 100 has a valve body 101 - which in the example described includes the portions 12a and 12b - which delimits a channel 102 for the passage of fluid that is intercepted by the obturator 22. The obturator can move between a closed condition of the channel 101 (figure 3A) and an open condition of the channel 102 (figure 3B). Thanks to the action of at least an elastic element (for example a spring) 21, the shutter is normally kept in the closed position of figure 3A. When, on the other hand, the insertion of the shank 15 pushes the obturator 22, overcoming the action of the elastic element 21, the obturator 22 can switch to the open condition of figure 3B where the channel 102 communicates with the passages 19 and 20 connecting the inside of the container 8 with the chamber 4.

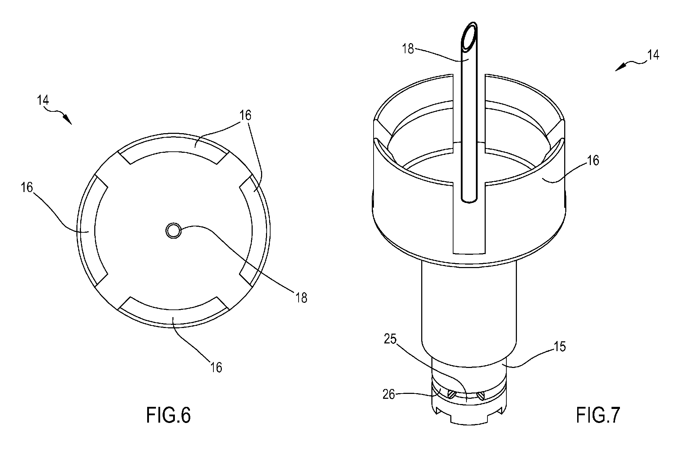

As can be seen from figure 7, the shank 15 of the transfer adapter 14 is made in such a way that when the shank is inserted in the tubular body there is no risk of contamination from the external environment nor risk of leakage of the material towards the external environment. For this purpose, the shank 15 is removably coupled to the tubular body and is displaceable up to the operative position, in which the shank acts to push the obturator thanks to a sliding seal between the radially external surface 15a of the shank and the radially internal surface 13a of the tubular body, which is located at the collar 13 of the ring nut 60.

More precisely, the radially external surface 15a of the shank 15 has a prismatic or cylindrical conformation, as in the case of example illustrated here, able to slidably engage with a radially inner surface 13a, also prismatic or cylindrical, of the tubular body 12. The radially external surface 15a defines at least an annular seating 25 in which an annular sealing element 25a operates, for example an O-ring made of elastomer material. Note that two or more annular seatings 25 can be present, axially offset and provided with respective sealing members 25a. Following insertion of the shank 15 in the tubular body 12, the sealing element 25a or sealing elements 25a are positioned between the radially external surface 15a of the shank and the radially internal surface 13a of the tubular body so that, as already mentioned, the shank 15 can be inserted and made to slide inside the tubular body while ensuring a fluid-tight and dust-tight seal.

On the opposite side to the shank 15, the transfer adapter 14 provides a coupling portion 16 capable of removably engaging with the end edge 10a of the container 8 housing the liquid component 9. As visible in figures 4-7, the coupling portion can be configured to define a snap fit with the edge 10a in such a way that as a result of the snap mechanism the perforation of the wall 11 is also facilitated. In accordance with an aspect of the invention, the portion 16 includes a plurality of axial elements 16a spaced angularly by slits 16b such as to define elastic teeth for constraining the edge 10a.

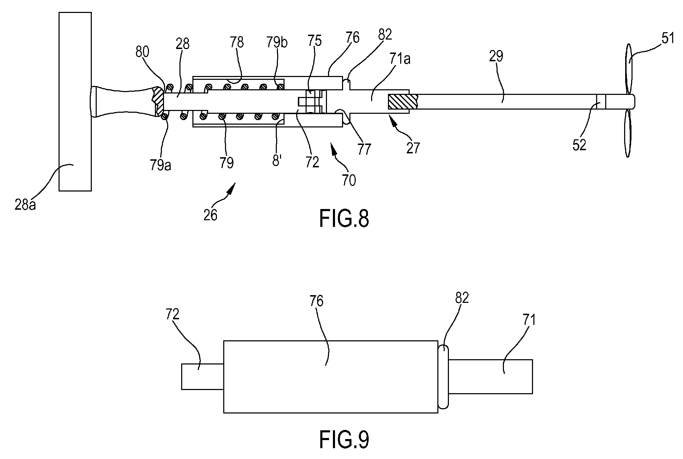

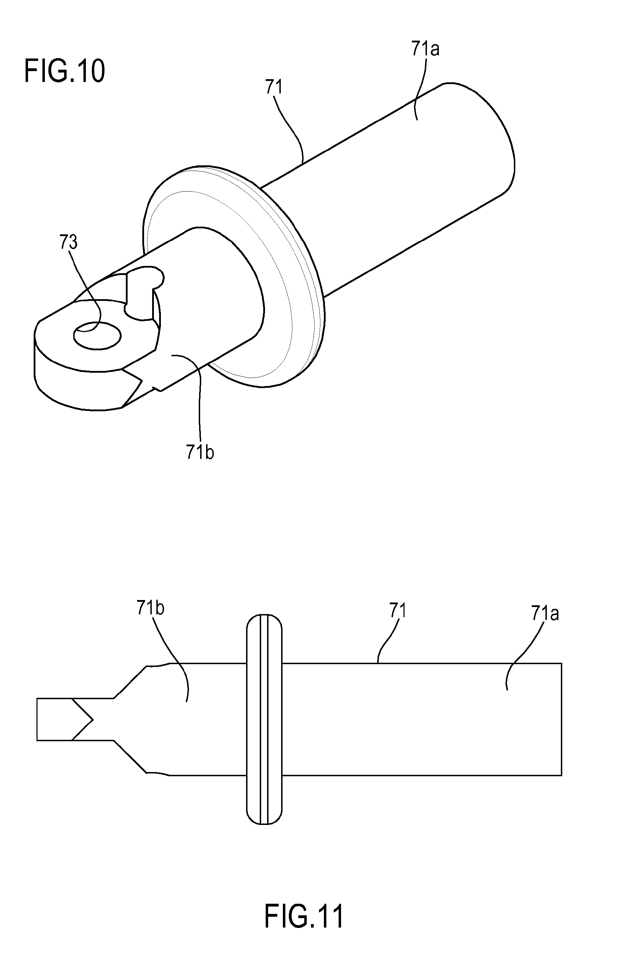

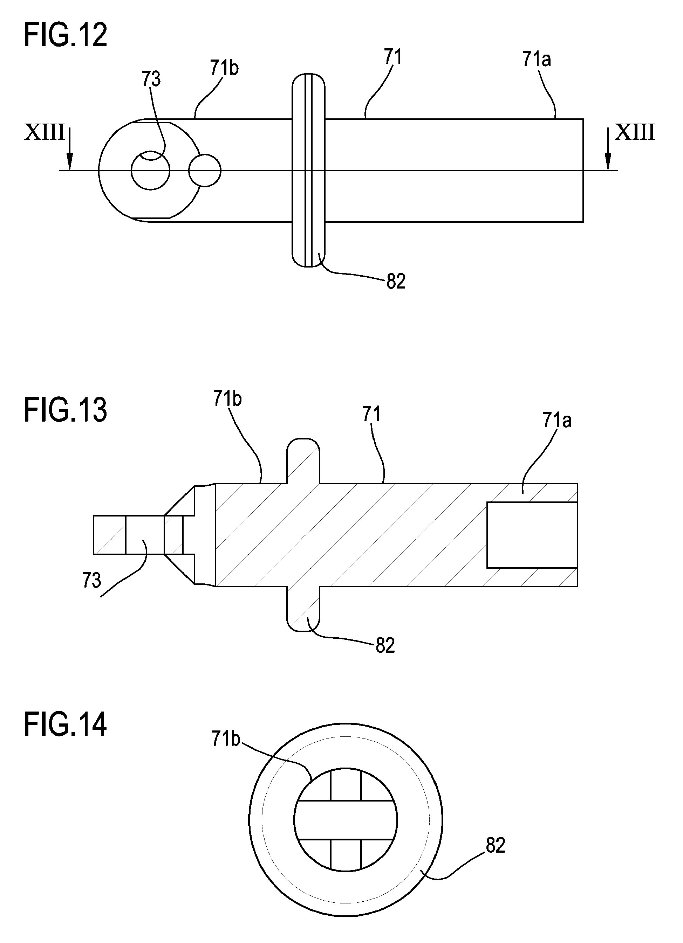

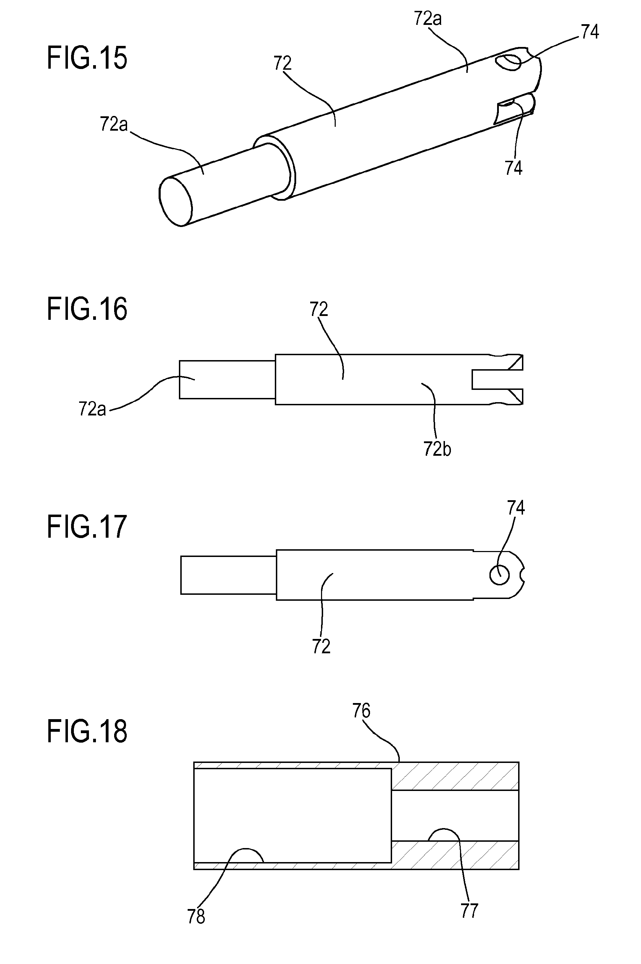

In accordance with a variant of the invention, the mixing member 26 has a stem 27 having an articulated joint 70 having a particular shape, as shown in figures 8-18. In this case in fact the articulated joint 70 comprises a first segment 71 and a second segment 72 engaged to one another. The first segment (see figures 10-14) has a first end 71a provided with an engagement portion connected to the active portion 29 and a second end 71b provided with a slot 73 which cooperates with one or more additional slots 74 afforded by the second end 72b of the second segment 72 and with a pin 75 to define a hinge, for example a flat hinge (see figures 15-17). The second segment 72 also exhibits a first end 72a connected and solidly constrained to the driving portion 28. As shown in figures 8 and 9 a stop element 76, for example shaped as a tubular sleeve, internally receives the coupling 50 when the coupling is to be maintained in the alignment condition of the two straight segments 71 and 72 and frees the coupling 50 when the first segment is to be angularly inclined with respect to the second segment. In particular, as shown in figures 8 and 18, the stop element or sleeve 76 has a first prismatic seating 77, i.e. cylindrical, complementarily shaped to the external surface of the segments 71 and 72 and a second prismatic seating 78, i.e. cylindrical, consecutive and of greater diameter than the first which houses the elastic element 79. The elastic element, for example comprising at least one helical spring, has a first end 79a acting in abutment against an edge 80 of, or solidly constrained to, a manoeuvring portion 28 and a second end 79b acting in abutment against a portion constrained to the stop element 76, for example in figure 8, the second end 79b of the spring acts in abutment against a connecting wall 81 between the first and the second prismatic, or cylindrical, seating. A radial lip 82 solidly constrained to the first segment and emerging from an outer lateral surface thereof delimits the axial travel of the sleeve with respect to the two segments: in practice, the spring 79 pushes the sleeve into the position shown in figure 8 and locks the rotation of the joint 70. By displacing the stop element 76 towards the manoeuvring portion 28 and overcoming the action of the spring the joint 70 can be released and then the two segments rotated in an inclined position thanks to the hinge defined by the slots 73, 74 and the pin 75 which allows to bend angularly an end part of the rod 27 with respect to an active portion 29 of the stem itself which is housed inside the chamber 4. As already described above, in the case of figure 8 too, the active portion 29 operating inside the chamber 4 can have one or more radial elements 51, for example blade-shaped which, by rotating the mixer 26, enable it to implement the mixing of the liquid component with the solid component. Note that since the housing body 3 is sealedly coupled both to the lid 34 and with the terminal element 38, and since the stem 27 passes through the through-opening 37 while still providing the fluid-tight seal, in the mixing step there is no risk that any liquid material and/or powders can come into contact with the user.

Finally, note that the stem 27 (both the one shown in figure 1 and the one of figure 8) can also comprise an easy-break segment 52 arranged in the vicinity of the radial elements 51. In practice, when at the end of mixing the manoeuvring portion is extracted from the chamber, the segment 52 is made to exit from the chamber 4 through the opening 37. Then, an inclination of the manoeuvring portion out of its own axis causes the breaking and the expansion of the segment 52 with the possibility therefore to eliminate all the manoeuvring part 28 and to proceed to the easier application of the container 3 to a dispenser device, such as a conventional pistol, which for example thrusts the piston 138 to cause the exit of cement from the port 6 where a conventional dispenser cannula can be applied.

### Materials used and construction of the Apparatus.

As has been briefly mentioned, the housing body may for example be formed in several parts, as shown for example in figure 1, namely it can comprise a tubular main body at ends of which the lid 34 and the element terminal 38 are fixed. Alternatively, instead of being formed in three parts, the housing body could be made in two parts or in a single piece: in terms of process the housing body can be realised by means of an injection moulding process, i.e. blow moulding or another type, such as to obtain a closed container with the relative access ports and outlet ports in several connected parts or in one piece.

The tubular body 12 can be removably engaged to the housing body 3, for example as illustrated in figure 2, using removable couplings that can be threaded, bayonet, snap or another type that enable selective engagement and disengagement of the tubular body 12 to the housing body 3. In an alternative technique, at the cover, the housing body 3 can be integral with the tubular body 12 in a single piece. In this case, the cover 34 can for example be realised in a separate piece with respect to the main body 33, such as to enable the assembly of the no-return valve internally of the tubular body 12.

With regard to the materials, the body of the housing is made of plastic material and in particular, at least the lateral wall of the housing body 3, i.e. in the example of figure 1, the main body 33, is made of typically transparent plastic material, such as to enable the operator to inspect the state of mixing of the liquid component with the solid component in the preparation step of the bone cement.

The adapter 14 is also realisable for example in plastic material, apart from the piercing element which can be either plastic or metal.

The container of the liquid component 8 can be made of glass or another transparent or translucent materials so that the level of the liquid content can be seen. The container is closed by a wall of perforable elastomer material, such as for example a plug of elastomer material, integral with the container or forced onto the container and secured by means of suitable retaining members such as, for example, a metal cap.

### Method for the formation of bone cements

In a further aspect of the invention, a process is provided for the formation of bone cements which can for example use an apparatus such as described herein above or in any case an apparatus according to one or more of the appended claims 1 to 18. The method includes the following steps.

After removing the mixing device 2 from its packaging, the chamber 4 of the mixing device 2 can be connected to a suction system able to generate a predetermined level of depression in the chamber 4. In this regard, it is sufficient to connect a suction line with the suction port 35 on the cover 34. If the mixing device 2 has a tubular body 12 with associated valve already integrated in the housing body, the transfer adapter is inserted at the free end of the tubular body 12. Conversely, if the tubular body is a separate component with respect to the housing body, the tubular body 12 is engaged to the housing body 3 at the input port 6 and then the transfer adapter is fitted into the free end 14 of the tubular body 12. The transfer adapter 14 can alternatively be already positioned in the tubular body during manufacturing, so that it is integral thereto, without, however, causing the opening of the inlet valve contained in the tubular body, opening that will be obtained only after the application of an axial force on the transfer adapter, as described in the following. The perforator 18 of the transfer adapter 14 can be covered by a protective cap, not shown, which, if present must be removed by the operator before proceeding to the next step.

The edge of the container 8 housing the liquid component is then inserted, possibly with a snap-fit, at the coupling portion 16 of the transfer adapter 14, thus determining the opening of a fluid passage through the wall and therefore through the outlet opening of the container 8. In practice, in the illustrated example the piercing element passes through the material of the wall and creates a fluid passageway through the wall. The container 8 of the liquid component is then pushed axially towards the transfer adapter 14, so that the shank 15 of the adapter can, in turn, push against the obturator 22 of the valve 100 by opening a fluid passage through the tubular body 12. In practice, the insertion of the shank 12 opens the valve 100, pushing the shutter away from the closed position, thereby allowing the fluid contained in the container 8 to cross the channel 20 inside the perforator 18, as well as the channel 19 inside the shank 15, and finally reach to inside the channel present in the tubular body 12, avoiding the obturator that has, incidentally, moved to its open position (figure 3B) and reaching the port 6, and then the chamber 4. If an aspirating line is connected, the passage of the fluid from the container 8 to the chamber 4 is also facilitated by the condition of partial vacuum created by the suction generated through the suction port 35 and by the presence of a predetermined amount of air or other gas in the container 8, which being positioned by gravity above the level of the contained liquid and expanding as a result of depression applied, pushes the liquid component towards the passage openings of the valve 100 contained in the tubular body 12, kept open by the pressure applied, and subsequently allows the liquid to flow into the chamber 4 of the mixing device. In addition to promoting the outflow of the liquid component, the condition of at least partial vacuum also improves the mixing of the liquid component with the solid component and enables degassing of the cement paste obtained.

After the valve has opened 100 the pushing condition of the container 8 towards the transfer adapter 14 has to be maintained for a short interval of time, depending on the volume of the liquid to be transferred and for example at least for a few seconds, allowing the liquid component to fully enter the chamber 4 of the mixing device. The condition of complete emptying of the container 8 is verified visually by the operator through the transparent wall thereof. Once this step has been completed, the container of the liquid component 8 and the transfer adapter 14 can be removed, which are made integral by the snap connection mechanism: the operator is then protected against sharp edges of the blade 18 which remains stuck in the elastomer wall 11 and inaccessible to external actions. The removal of the assembly of the container 8 and the adapter 14 from the tubular body 12 enables the elastic return of the spring acting on the obturator 22 and thus the closure of the valve 100. In these conditions the chamber 4 is isolated from the external environment and the operator can proceed to mix the liquid component with the solid component without risk of contamination of components and without the possibility of leakage of dust and/or liquids. The mixing proceeds until the cement paste is formed and then the paste is dispensed in the site provided for prosthetic cementing, i.e. the bone consolidation.

Before the actual dispensing the following steps can be included. At the end of mixing, after having disconnected the vacuum line if used, the driving portion 38 is extracted from the chamber 4: in this way, the easy-break segment 52 is made to exit from the chamber 4 through the opening 37. Then, by inclining the manoeuvring portion out of its own axis, the segment 52 will break and expand and all the manoeuvring part 28 will be removed. The valved tubular body 12 is then removed from the valved port 6 to which a cannula dispenser is applied. At this point the container 3 can be coupled to a conventional pistol, which for example acts to thrust the piston 138 to promote the escape of cement from the door 6.

## Claims

1. An apparatus for preparation of bone cements (1), comprising:
a mixer device (2) having:
a housing body (3) defining a chamber (4), housing or able to house a predetermined quantity of a solid component (5), for example in a granular or powder form,
at least an inlet port (6) for enabling access of at least a fluid towards the chamber (4);
a check and closing valve (7) operating in proximity of or at the inlet port (6), the check and closing valve (100) comprising a valve body (101) defining a fluid passage channel (102) and an obturator organ (22) cooperating with the valve body (101) and displaceable between a closed condition, in which it intercepts and blocks the passage of fluid through the channel (102), and an open condition, in which it enables passage of fluid through the channel (102), the valve (100) being predisposed to selectively open and close passage of fluid towards the chamber (4) of the housing body (3).

2. The apparatus of claim 1, further comprising at least a container (8) housing a predetermined quantity of a liquid component (9) and comprising at least an outlet opening (10) at which a closing organ (11) operates; and at least a transfer adapter (14) removably engageable, on a side, to the container (8), and, on an opposite side, to the inlet port (6); the transfer adapter, when engaged to the container (8) and the port (6), acting to open both the closing organ (11) and to push the obturator (22), opening the valve (100).

3. The apparatus of claim 1 or 2, comprising a tubular body (12) defining the valve body (7) and having a first terminal portion (12a) engaged to the inlet port (6) and a second terminal portion (12b) facing away from the housing body (3) of the mixer device (2) and defining an engaging collar (13).

4. The apparatus of claim 2 or 3, wherein the transfer adapter (14) has a shank (15) able to slidably engage with the valve body (101), optionally with the engaging collar (13) of the tubular body, and to displace in the valve body up to an operating position in which the shank (15) acts to open the valve; and
wherein the transfer adapter (14) comprises, in an axially opposite position to the shank (15), a removable coupling portion (16) with the container (8) of the liquid component, the coupling portion (16) being configured such as to act on the closing organ (11) and to determine the opening of the fluid passage through the outlet opening (10) when the coupling portion (16) engages with the container (8) of the liquid component (9).

5. The apparatus of claim 4, wherein the closing organ (11) comprises a perforable membrane (17) sealing the outlet opening of the container of the liquid component, and wherein the coupling portion (16) of the transfer adapter (14) comprises a piercing element (18) able to pass through the perforable membrane (17) when the coupling portion engages with the container (8) of the liquid component;
and wherein the shank (15) comprises an internal channel (19) axially crossing the shank and in communication with a further channel (20) crossing the coupling portion (16), in particular internally crossing the piercing element.

6. The apparatus of any one of the preceding claims, wherein the valve (100) comprises an elastic thrust element (21) housed in the tubular body and active on the obturator (22) which is destined to operate normally to close the fluid passage across the valve body acting in abutment, by a thrust of the elastic element, against a seal edge (23) predisposed on an internal surface of the valve body (101).

7. The apparatus of claim 6, wherein the elastic element (21), for example comprising at least a helix spring, exhibits a first end (21a) that acts on the obturator (22) and a second end (21b) that acts on an abutment (24) that is axially solid with the tubular valve body (101), the elastic element (21) being normally compressed such as to exert an elastic thrust action on the obturator that is sufficient to maintain the obturator in the closed position even in the presence of a difference between the pressure existing internally of the chamber (4) and the ambient atmospheric pressure existing externally of the housing body (3) of greater than 0.3 bar, or optionally greater than 0.5 bar, or even more optionally greater than 0.8 bar.

8. The apparatus of any one of the preceding claims from 4 to 7, wherein the shank (15) of the transfer adapter (14) is removably couplable to the valve body, in particular the tubular body (12), and is displaceable up to the operating position in which the shank (15) acts to push the obturator (22), opening a fluid passage between the internal channel (20) of the piercing element and the channel (19) crossing the shank and thus setting the internal channel (20) and the piercing element in communication with the inlet port (6) of the chamber (4) of the mixer device.

9. The apparatus of any one of claims from 4 to 8, wherein the shank (15) comprises a radially external surface (15a) of prismatic conformation, i.e. cylindrical, capable of fluid-sealedly slidably joining with an internal surface (12c) of the valve body (12), in particular with an internal surface (12c) of the tubular body (12), the radially external surface (15a) defining at least an annular seating (25) in which a seal element (25a), also having an annular conformation, operates, the seal element (25a) being positioned between the radially external surface of the shank and the radially internal surface of the valve body when the shank is inserted and made to slide in the valve body.

10. The apparatus of any one of claims from 2 to 9, wherein the container (8) of the liquid component (9) comprises a containing element having an at least partially deformable portion, for example axially deformable, in such a way that the internal housing volume of the liquid component (9) can progressively be reduced as the liquid component is extracted from the container (8) and wherein the container (8) comprises one of the following:
- a containing element constituted by a bag made of a plastic material which progressively collapses on itself as the liquid component is extracted,
- a containing element constituted by a bag made of a plastic material having a bellows portion that is progressively axially collapsible as the liquid is extracted,
- a containing element having a lateral wall and a terminal wall, opposite the opening, and slidable axially and sealedly along the lateral wall progressively nearingly to the opening as the liquid component is extracted,
- a syringe having a sealedly slidable piston internally of a syringe body that on an opposite side with respect to the piston is configured such as to directly or indirectly engage to the valve body.

11. The apparatus of any one of the preceding claims, wherein the mixer device exhibits at least a mixer organ (26) having a stem (27) provided with a manoeuvring portion (28) emerging externally of the housing body and an active portion (29) internally housed in the chamber (4), and having one or more radial mixer elements (30); and wherein the manoeuvring portion (28) comprises at least a joint (31) for enabling angularly bending a terminal part of the stem with respect to the active portion thereof, the terminal part further optionally bearing a gripping handle (32).

12. The apparatus of any one of the preceding claims, wherein the housing body (3) comprises a tubular main body (33) having an end to which a cover (34) is sealedly fixed exhibiting in turn the inlet port (6), an aspirating port (35) distinct from the inlet port and connectable to a vacuum line and a through-opening (37) such as to receive a stem (27) of a mixer organ (26),
optionally wherein the housing body (3), on the opposite side to the cover (34), comprises a terminal closing element (38) fixed sealedly to the main body,
optionally wherein the housing body (3) is made in a plastic material, for example an optically transparent or translucent plastic material.

13. The apparatus of any one of the preceding claims, wherein the valve body, optionally the tubular body (12), is unremovably joined to the inlet port.

14. An apparatus for preparing bone cements, optionally according to any one of the preceding claims, comprising a mixer device (2) having:
a housing body (3) defining a chamber (4) housing or able to house a predetermined quantity of a solid component (5), for example in a granular or a powder form,
at least an inlet port (6) for enabling fluid access towards the chamber (4);
wherein the mixer device exhibits at least a mixer organ (26) having:
a stem (27) exhibiting a manoeuvring portion (28) emerging from the housing body and an active portion (29) housed internally of the chamber (4),
a joint (70) comprising a first segment (71) and a second segment (72) rotatably engaged to one another, and wherein the first segment has a first end (71a) connected to the active portion (29) and
a second end (71b) cooperating with the second end (72b) of the second segment (72) such as to define a hinge, for example a flat hinge, the second segment (72) being connected to the manoeuvring portion (28), and
a stop element (76), for example shaped as a tubular sleeve, which in a first operating position internally receives the joint (50) and maintains the two segments (71 and 72) in the alignment condition and in a second operating condition distanced axially or angularly with respect to the first operating position, frees the joint (50) and enables angularly inclining the first segment with respect to the second segment;
optionally wherein the sleeve (76) exhibits a first prismatic or cylindrical seating (77) complementarily shaped to the external surface of the two segments (71 and 72) and a second prismatic or cylindrical seating (78) consecutive to and having a greater diameter than the first,
which second sliding seating houses an elastic element (79) which in turn exhibits a first end (79a) acting abuttingly against an edge (80) solidly constrained to the manoeuvring portion (28) and a second end (79b) acting abuttingly against a connecting wall (81) between the first and the second seating; and wherein a radial lip (82) solidly constrained to the first segment and emerging from an external lateral surface thereof delimits the axial travel of the sleeve with respect to the two segments.

15. A process for forming bone cements using an apparatus as in any one of the preceding claims, comprising steps of:
a. connecting the chamber (4) of the mixer device (2) to an aspirating system for generating a predetermined depression level in the chamber;
b. in a case in which the valve body (12) is a separate component from the inlet port (6), engaging the valve body to the inlet port;
c. coupling the transfer adapter (14) to the free end of the valve body;
d. inserting at least the portion of the container (8) of the liquid component delimiting the outlet opening at the coupling portion (16) of the transfer adapter (14) such as to determine an opening of a passage of fluid through the outlet opening;
e. axially pushing the container (8) of the liquid component towards the transfer adapter (14), in such a way that the shank (15) of the adapter can, in turn, act to push on the obturator (22) of the valve (100) and thus open a fluid passage from the container of the liquid component up to the chamber of the mixer device;
f. maintaining the pushed condition for a predetermined time, for example at least 10 seconds, enabling the liquid component to enter the chamber (4) of the mixer device containing the solid component;
g. removing the container (8) of the liquid component and the transfer adapter (14) from the tubular body (12) such as to enable the valve (100) to be closed;
h. mixing for a predetermined time, up to formation of the bone cement.
